# EUROPEAN PATENT APPLICATION

(11) **EP 4 194 082 A1**
(43) Date of publication of application: **14.06.2023**
(21) Application number: 22862358.3
(22) Date of filing: 25.07.2022
(51) Int. Cl.: B01J 8/00

(54) **SYSTEM FOR TRANSFERRING REACTION SOLUTION**

(30) Priority: 08.10.2021 KR 20210134020; 01.07.2022 KR 20220081412
(71) Applicant: LG CHEM, LTD., Yeongdeungpo-gu, Seoul 07336 (KR)
(72) Inventor: YOUK, Kyung Seog, Daejeon 34122 (KR); SHIN, Joon Ho, Daejeon 34122 (KR); SONG, Jong Hun, Daejeon 34122 (KR); LEE, Jeong Seok, Daejeon 34122 (KR); LEE, Hong Min, Daejeon 34122 (KR); HWANG, Moon Sub, Daejeon 34122 (KR)
(74) Representative: Hoffmann Eitle
(86) International application number: PCT/KR2022/010917
(87) International publication number: WO 2023/058866

(57) **Abstract**

Provided is a system for transferring reaction solution, including: a reactor that receives and reacts a feed stream to form a reaction solution; a reactor discharge pipe that is provided on a side portion of the reactor at a position corresponding to a surface height of the reaction solution in the reactor and connected to a precipitation tank to transfer the reaction solution from the reactor to the precipitation tank; and a precipitation tank that precipitates a polymer contained in the reaction solution.

## Description

### [Technical Field]

### CROSS-REFERENCE TO RELATED APPLICATIONS

The present application claims the benefit of priority to Korean Patent Application No. 10-2021-0134020, filed on October 8, 2021, and No. 10-2022-0081412, filed on July 1, 2022, the entire contents of which are incorporated herein as a part of the specification.

### Technical Field

The present invention relates to a system for transferring reaction solution, and more particularly, to a method for smoothly transferring a reaction solution in an ethylene oligomerization reactor.

### [Background Art]

Alpha olefin is widely used commercially as an important material used as a comonomer, a detergent, a lubricant, a plasticizer, and the like. In particular, 1-hexene and 1-octene have been widely used as a comonomer for controlling a density of polyethylene in a production of linear low-density polyethylene (LLDPE).

Alpha olefins such as 1-hexene and 1-octene are typically prepared through oligomerization of ethylene. The oligomerization reaction of ethylene is performed by the oligomerization reaction (trimerization reaction or tetramerization reaction) of ethylene in the presence of a catalyst using ethylene as a reactant. Through the reaction, a product containing the desired 1-hexene and 1-octene is produced, and a small amount of polymer may be produced as a by-product during the catalytic reaction. A polymer floating in the reaction solution causes a fouling phenomenon in a control valve for keeping a height of a reaction solution in the reactor constant in the process of transferring the reaction solution to a storage container using a reactor discharge pipe, and as a result, an operation of the reactor need to stop and pipe and valves need to be washed.

### [Disclosure]

### [Technical Problem]

The present invention provides a method of smoothly transporting a reaction solution by preventing a control valve from clogging due to a polymer contained in a reaction solution discharged from an ethylene oligomerization reactor.

### [Technical Solution]

In one general aspect, a system for transferring reaction solution includes: a reactor that receives and reacts a feed stream to form a reaction solution; a reactor discharge pipe that is provided on a side portion of the reactor at a position corresponding to a surface height of the reaction solution in the reactor and connected to a precipitation tank to transfer the reaction solution from the reactor to the precipitation tank; and a precipitation tank that precipitates a polymer contained in the reaction solution.

### [Advantageous Effects]

According to a method of washing a reactor of the present invention, by designing a pipe in such a structure that a reaction solution may overflow in the reactor and by disposing a precipitation tank between the reactor and a storage container to remove a polymer contained in the reaction solution and then transferring the reaction solution to the storage container through a precipitation tank discharge pipe provided with a control valve, it is possible to prevent a fouling phenomenon caused by the polymer contained in the reaction solution from occurring to smoothly transfer the reaction solution.

In addition, by using the precipitation tank to remove the polymer contained in the reaction solution, it is possible to reduce the energy cost and time for removing the polymer from the reaction solution at a downstream and for separating products.

### [BRIEF DESCRIPTION OF THE DRAWINGS]

FIG. 1 is a process flow diagram of a system for transferring reaction solution according to an embodiment of the present invention.
FIG. 2 is a process flow diagram of a system for transferring reaction solution according to a Comparative Example.

### [Best Mode]

Terms and words used in the present specification and claims are not to be construed as a general or dictionary meaning but are to be construed as meaning and concepts meeting the technical ideas of the present invention based on a principle that the inventors can appropriately define the concepts of terms in order to describe their own inventions in best mode.

In the present invention, the term 'stream' may mean a flow of a fluid in a process, and may also mean a fluid itself flowing in a moving line (pipe). Specifically, the 'stream' may mean both the fluid itself and the flow of the fluid flowing within the pipe connecting each device. In addition, the fluid may include any at least one or more components of a gas, a liquid, and a solid.

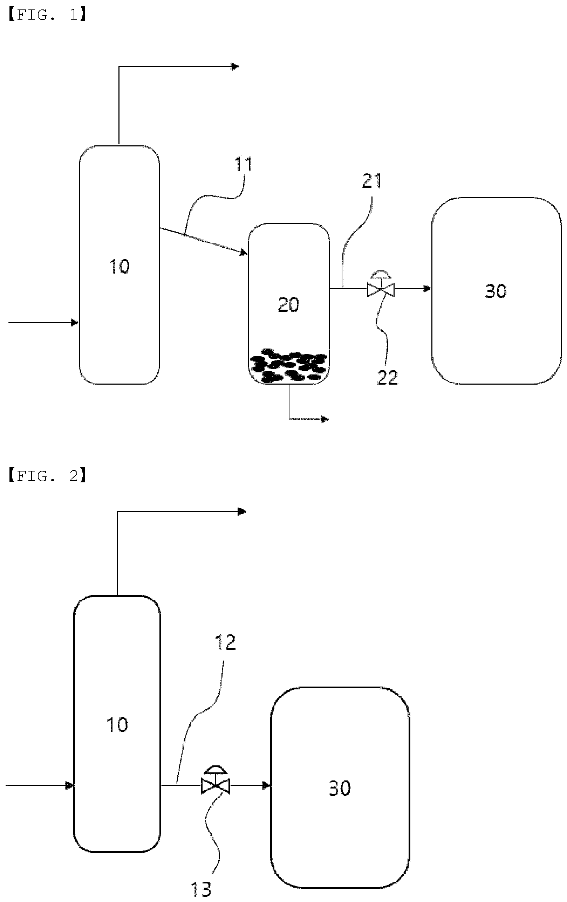

Hereinafter, the present invention will be described in more detail with reference to FIG. 1 to help the understanding of the present invention.

According to the present invention, a system for transferring reaction solution is provided. The system for transferring reaction solution includes: a reactor 10 that receives and reacts a feed stream to form a reaction solution; a reactor discharge pipe 11 that is provided on a side portion of the reactor 10 at a position corresponding to a surface height of the reaction solution in the reactor 10 and connected to a precipitation tank 20 to transfer the reaction solution from the reactor 10 to the precipitation tank 20; and a precipitation tank 20 that precipitates a polymer contained in the reaction solution.

According to an embodiment of the present invention, the type of the reactor 10 and the type of reaction occurring in the reactor 10 are not particularly limited, and when a polymer capable of causing a fouling phenomenon is included in the reaction solution through the reaction occurring in the reactor 10, the system for transferring reaction solution according to the present invention may be applied without limitations.

The reactor 10 may be, for example, an ethylene oligomerization reactor. Specifically, alpha olefin is widely used commercially as an important material used as a comonomer, a detergent, a lubricant, a plasticizer, and the like. In particular, 1-hexene and 1-octene have been widely used as a comonomer for controlling a density of polyethylene in a production of linear low-density polyethylene (LLDPE), and the alpha olefin may be prepared through oligomerization reaction of ethylene.

The oligomerization reaction of ethylene may be performed by a trimerization reaction or a tetramerization reaction of ethylene in the presence of a catalyst using ethylene as a reactant.

The oligomerization reaction may refer to a reaction in which a monomer is polymerized. Depending on the number of monomers to be polymerized, trimerization and tetramerization are called, and these trimerization and tetramerization are collectively called multimerization.

A feed stream supplied to the reactor 10 may include, for example, ethylene as a reactant, a solvent, a catalyst, a co-catalyst, and the like.

The catalyst used for the oligomerization reaction of ethylene may include a transition metal supply source. The transition metal supply source may be a compound containing one or more selected from the group consisting of, for example, chromium(III) acetylacetonate, chromium(III) chloride tetrahydrofuran, chromium(III) 2-ethylhexanoate, chromium(III) tris(2,2,6,6-tetramethyl-3,5-heptanedionate), chromium (III) benzoylacetonate, chromium (III) hexafluoro-2,4-pentaindionate, chromium (III) acetate hydroxide, chromium (III) acetate, chromium(III) butyrate, chromium(III) pentanoate, chromium(III) laurate, and chromium(III) stearate.

The co-catalyst may include one or more selected from the group consisting of, for example, trimethyl aluminum, triethyl aluminum, triisopropyl aluminum, triisobutyl aluminum, ethyl aluminum sesquichloride, diethylaluminum chloride, ethyl aluminum dichloride, methylaluminoxane, modified methylaluminoxane, and borate.

For example, the solvent may include one or more selected from the group consisting of n-pentane, n-hexane, n-heptane, cyclohexane, methyl cyclohexane, octane, cyclooctane, decane, dodecane, benzene, xylene, 1,3,5-trimethylbenzene, toluene, ethylbenzene, chlorobenzene, dichlorobenzene, and trichlorobenzene.

As such, the reaction solution is formed in the process of oligomerizing an ethylene monomer in the presence of the catalyst, and in the reaction solution, in addition to the oligomer product, a polymer such as polyethylene may be produced as a by-product during the catalytic reaction. In the process of transferring the reaction solution to the storage container using a reactor discharge pipe, the polymer floating in the reaction solution is severely fouled in the control valve for keeping the height of the reaction solution in the reactor constant to prevent the reaction solution from being discharged, so the height of the reaction solution gradually rises. As a result, the operation of the reactor abnormally stops and the pipes and valves need to be washed.

Conventionally, a lot of time was taken in the process of dismantling the pipe and control valve, washing them for a long time, and then reinstalling them in order to wash the control valve fouled due to the polymer, causing a problem in that production is reduced and cleaning cost is increased when the operation is shut down, and reaction stability was deteriorated due to an increase in a reaction normalization time after washing.

In addition, there is a problem in that an additional separation column needs to be operated in order to separate and remove the polymer contained in the reaction solution at a downstream, and when the content of the polymer contained in the reaction solution is high, the energy cost and time for separating and removing the polymer increase.

On the other hand, according to the present invention, by designing the pipe so that the reaction solution overflows from the reactor and by transferring the reaction solution to the precipitation tank to precipitate and remove the polymer, and then transferring the reaction solution to the storage container through a precipitation tank discharge pipe provided with the control valve to prevent the occurrence of fouling in the control valve due to the polymer contained in the reaction solution, it is possible to smoothly transfer the reaction solution.

In addition, by using the precipitation tank to remove the polymer contained in the reaction solution, it is possible to reduce the energy cost and time for removing the polymer from the reaction solution at a downstream and for separating products.

According to the embodiment of the present invention, the system for transferring reaction solution may further include the reactor discharge pipe 11 that is provided in a side portion of the reactor 10 at a position corresponding to the surface height of the reaction solution in the reactor 10, and is connected to the precipitation tank 20 to transfer the reaction solution in the reactor 10 to the precipitation tank 20.

According to the progress of the reaction in the reactor 10, the surface height of the reaction solution may increase as a certain amount of products is generated. Therefore, the surface height of the reaction solution to be maintained may vary depending on the type of desired products, process conditions, etc., and accordingly, the height at which the reactor discharge pipe 11 is installed may be adjusted.

The feed stream may be supplied from the reactor 10 and reacted to form the reaction solution containing the product, and the unreacted reactant may be discharged to an upper portion of the reactor 10, and may be recovered and reused in a downstream process.

The reactor discharge pipe 11 may be connected from the side portion of the reactor 10 at the position corresponding to the surface height of the reaction solution in the reactor 10 to the precipitation tank 20. In this case, the reactor discharge pipe 11 may be provided to be inclined downward from the reactor 10 to the precipitation tank 20. By designing the reactor discharge pipe 11 in this way, when the reaction solution reaches a certain height in the reactor 10, the reaction solution may overflow through the reactor discharge pipe 11 to be transferred to the precipitation tank 20.

The slope of the reactor discharge pipe 11 extending from the reactor 10 may be, for example, 5° or more, 7° or more or 8° or more and 10° or less, 15° or less, 20° or less, or 30° or less. By designing the reactor discharge pipe 11 to have a downward slope in the direction of the precipitation tank 20 at such a slope, the reaction solution is discharged from the reactor 10 through the reactor discharge pipe 11, and may be transferred smoothly to the precipitation tank 20.

In addition, the reactor discharge pipe 11 may not include the control valve. Specifically, by not installing the control valve in the reactor discharge pipe 11, it is possible to prevent the occurrence of the fouling phenomenon in which the control valve is clogged due to the polymer contained in the reaction solution transferred through the reactor discharge pipe 11.

The operating pressure of the reactor 10 may be the same as the operating pressure of the precipitation tank 20. By maintaining the operating pressure of the reactor 10 and the operating pressure of the precipitation tank 20 at the same pressure, the reaction solution in the reactor 10 may overflow through the reactor discharge pipe 11 to be transferred to the precipitation tank 20. On the other hand, when the operating pressure of the reactor 10 is lower than the operating pressure of the precipitation tank 20, the reaction solution is not smoothly discharged, so there is a problem in that the height of the reaction solution in the reactor 10 rises, and when the operating pressure of the reactor 10 is higher than a pressure of the precipitation tank 20, there is a problem in that the flow of the unreacted reactant becomes unstable.

According to an embodiment of the present invention, the reaction solution may be transferred to the precipitation tank 20 through the reactor discharge pipe 11, and the polymer contained in the reaction solution may be precipitated and removed in the precipitation tank 20.

The operating pressure of the precipitation tank 20 may vary depending on the operating pressure of the reactor 10. For example, the operating pressure of the precipitation tank 20 may be 20 kg/cm².g or more, 25 kg/cm².g or more or 30 kg/cm².g or more and 40 kg/cm².g or less, 45 kg/cm².g or less, or 50 kg/cm².g or less.

The operating temperature of the precipitation tank 20 may be appropriately adjusted to precipitate and remove the polymer contained in the reaction solution. For example, the operating temperature of the precipitation tank 20 may be 10°C or higher, 15°C or higher or 20°C or higher and 70°C or lower, 80°C or lower, or 90°C or lower.

In this way, by controlling the operating conditions of the precipitation tank 20, the polymer included in the reaction solution in the precipitation tank 20 may be effectively removed, so the energy cost for separating the product from the reaction solution at the downstream may be saved and the time may be shortened. In this case, the polymer may have a weight average molecular weight of 80,000 g/mol to 300,000 g/mol, 100,000 g/mol to 200,000 g/mol, or 100,000 g/mol to 150,000 g/mol.
a polymer precipitated in a solid state and a supernatant containing a reaction solution from which the polymer is removed may be formed in the precipitation tank 20. Specifically, the supernatant containing the reaction solution from which the polymer is removed may be transferred to the downstream process. In this case, it is possible to save the energy cost and time for separation in the step of separating the product of the downstream by removing the polymer in advance from the precipitation tank 20.

A precipitation tank discharge pipe 21 may be provided in the side portion of the precipitation tank 20 at the height at which the supernatant is formed. Specifically, the precipitation tank discharge pipe 21 is formed in the side portion of the precipitation tank 20 at the height in which the supernatant is formed in the precipitation tank 20 to discharge the reaction solution from which the polymer is removed from the precipitation tank 20 and transfer the reaction solution.

The precipitation tank discharge pipe 21 may include a control valve 22. Specifically, the control valve 22 may control the flow rate of the reaction solution discharged from the precipitation tank 20, and further maintain the height of the reaction solution in the reactor 10.

The precipitation tank discharge pipe 21 may be provided at a position lower than the height at which the reactor discharge pipe 11 is connected to the precipitation tank 20. Specifically, by providing the precipitation tank discharge pipe 21 at the position lower than the height at which the reactor discharge pipe 11 is connected to the precipitation tank 20, it is possible to prevent the reaction solution from which the polymer is removed in the precipitation tank 20 from reflowing into a reactor 10, and by allowing the reaction solution to overflow smoothly from the reactor 10 to the precipitation tank 20, it is possible to maintain the height of the reaction solution in the reactor 10.

According to an embodiment of the present invention, the system for transferring reaction solution may further include a storage container 30 for receiving and storing the reaction solution from the precipitation tank 20. The storage container 30 may be connected to the precipitation tank 20 through the precipitation tank discharge pipe 21.

The storage container 30 may receive and store the reaction solution from which the polymer is removed from the precipitation tank 20, and may control the transfer to the downstream process of separating the product.

The pressure of the storage container 30 may be lower than the pressure of the precipitation tank 20. For example, the pressure of the storage container 30 is 1 kg/cm².g or more, 1.5 kg/cm².g or more or 2 kg/cm².g or more and 4 kg/cm².g or less, 4.5 kg/cm².g or less, or 5 kg/cm².g or less that lower than the pressure of the precipitation tank 20. In this way, by adjusting the pressure of the storage container 30 to be lower than the pressure of the precipitation tank 20, the reaction solution may be transferred from the precipitation tank 20 to the storage container 30 using the pressure difference. In this case, the reaction solution transferred from the precipitation tank 20 to the storage container 30 is transferred through the precipitation tank discharge pipe 21 provided with the control valve 22, and by removing the polymer in the reaction solution in the precipitation tank 20, it is possible to prevent the occurrence of the fouling phenomenon in which the control valve is clogged due to the polymer during the transfer.

Hereinabove, the system for transferring reaction solution according to the present invention has been shown in the description and drawings, but the above drawings and the description describe and illustrate only the essential components for understanding the present invention. In addition to the processes and apparatus illustrated in the above description and drawings, processes and apparatus not separately described and shown may be appropriately applied and used to implement the system for transferring reaction solution according to the present invention.

Hereinafter, the present invention will be described in more detail with through Examples. However, the following examples are for illustrating the present invention, and it is clear to those skilled in the art that various changes and modifications are possible within the scope and spirit of the present invention, and the scope of the present invention is not limited only thereto.

### Examples

### Example 1

A reaction solution was transferred according to a process flow diagram illustrated in FIG. 1 below.

An oligomerization reaction of ethylene was performed by supplying a feed stream to a reactor 10, which was identically performed in Examples 2 to 4 and Comparative Example 1 below.

In order to maintain a height of the reaction solution in the reactor 10 at 1.4 m, a reactor discharge pipe 11 is installed in a side portion of the reactor 10 at a position corresponding to a surface height of the reaction solution, and thus, the reaction solution overflowed into a precipitation tank 20 through the reactor discharge pipe 11. In this case, the reactor discharge pipe 11 was connected to the precipitation tank 20, had a downward slope of 10° in the direction of the precipitation tank 20, and was not provided with a control valve. In addition, operating pressures of the reactor 10 and the precipitation tank 20 were equally controlled to be 30 kg/cm².g.

Under the condition of controlling a temperature of the precipitation tank 20 to 20°C, the polymer in the reaction solution was precipitated in a solid state and removed, and a supernatant containing the reaction solution from which the polymer was removed was formed. A precipitation tank discharge pipe 21 was installed at a height at which the supernatant was formed, and the reaction solution from which the polymer was removed was transferred to a storage container 30 through the precipitation tank discharge pipe 21. In this case, the precipitation tank discharge pipe 21 was installed at a position lower than the height of the reactor discharge pipe 11 connected to the precipitation tank 20, and the precipitation tank discharge pipe 21 was provided with the control valve 22. In addition, the operating pressure of the storage container 30 was controlled to be lower than the operating pressure of the precipitation tank 20 to be 27 kg/cm².g.

This operation was performed for 10 hours, and after 10 hours operation, the height of the reaction solution in the reactor 10 was measured. As a result, it could be confirmed that the height of the reaction solution in the reactor 10 was maintained at 1.4 m. This may mean that the control valve 22 provided in the precipitation tank discharge pipe 21 is not clogged by the polymer, and thus, the reaction solution is smoothly discharged from the reactor 10 and the height of the reaction solution is maintained. In this case, it could be confirmed that it is possible to continue the operation.

### Example 2

A reaction solution was transferred according to a process flow diagram illustrated in FIG. 1 below.

An oligomerization reaction of ethylene was performed by supplying a feed stream to the reactor 10.

In order to maintain a height of the reaction solution in the reactor 10 at 1.4 m, a reactor discharge pipe 11 is installed in a side portion of the reactor 10 at a position corresponding to a surface height of the reaction solution, and thus, the reaction solution overflowed into the precipitation tank 20 through the reactor discharge pipe 11. In this case, the reactor discharge pipe 11 was connected to the precipitation tank 20, had a downward slope of 10° in the direction of the precipitation tank 20, and was not provided with a control valve. In addition, operating pressures of the reactor 10 and the precipitation tank 20 were equally controlled to be 30 kg/cm².g.

Under the condition of controlling a temperature of the precipitation tank 20 to 50°C, the polymer contained in the reaction solution was precipitated in a solid state and removed, and a supernatant containing the reaction solution from which the polymer was removed was formed. A precipitation tank discharge pipe 21 was installed at a height at which the supernatant was formed, and the reaction solution from which the polymer was removed was transferred to a storage container 30 through the precipitation tank discharge pipe 21. In this case, the precipitation tank discharge pipe 21 was installed at a position lower than the height of the reactor discharge pipe 11 connected to the precipitation tank 20, and the precipitation tank discharge pipe 21 was provided with the control valve 22. In addition, the operating pressure of the storage container 30 was controlled to be lower than the operating pressure of the precipitation tank 20 to be 27 kg/cm².g.

This operation was performed for 10 hours, and after 10 hours operation, the height of the reaction solution in the reactor 10 was measured. As a result, it could be confirmed that the height of the reaction solution in the reactor 10 was maintained at 1.4 m. This may mean that the control valve 22 provided in the precipitation tank discharge pipe 21 is not clogged by the polymer, and thus, the reaction solution is smoothly discharged from the reactor 10 and the height of the reaction solution is maintained. In this case, it could be confirmed that it is possible to continue the operation.

### Example 3

A reaction solution was transferred according to a process flow diagram illustrated in FIG. 1 below.

An oligomerization reaction of ethylene was performed by supplying a feed stream to the reactor 10.

In order to maintain a height of the reaction solution in the reactor 10 at 1.4 m, a reactor discharge pipe 11 is installed in a side portion of the reactor 10 at a position corresponding to a surface height of the reaction solution, and thus, the reaction solution overflowed into the precipitation tank 20 through the reactor discharge pipe 11. In this case, the reactor discharge pipe 11 was connected to the precipitation tank 20, had a downward slope of 10° in the direction of the precipitation tank 20, and was not provided with a control valve. In addition, operating pressures of the reactor 10 and the precipitation tank 20 were equally controlled to be 30 kg/cm².g.

Under the condition of controlling a temperature of the precipitation tank 20 to 70°C, the polymer contained in the reaction solution was precipitated in a solid state and removed, and a supernatant containing the reaction solution from which the polymer was removed was formed. A precipitation tank discharge pipe 21 was installed at a height at which the supernatant was formed, and the reaction solution from which the polymer was removed was transferred to a storage container 30 through the precipitation tank discharge pipe 21. In this case, the precipitation tank discharge pipe 21 was installed at a position lower than the height of the reactor discharge pipe 11 connected to the precipitation tank 20, and the precipitation tank discharge pipe 21 was provided with the control valve 22. In addition, the operating pressure of the storage container 30 was controlled to be lower than the operating pressure of the precipitation tank 20 to be 27 kg/cm².g.

This operation was performed for 10 hours, and after 10 hours operation, the height of the reaction solution in the reactor 10 was measured. As a result, it could be confirmed that the height of the reaction solution in the reactor 10 was maintained at 1.4 m. This may mean that the control valve 22 provided in the precipitation tank discharge pipe 21 is not clogged by the polymer, and thus, the reaction solution is smoothly discharged from the reactor 10 and the height of the reaction solution is maintained. In this case, it could be confirmed that it is possible to continue the operation.

### Example 4

A reaction solution was transferred according to a process flow diagram illustrated in FIG. 1 below.

An oligomerization reaction of ethylene was performed by supplying a feed stream to the reactor 10.

In order to maintain a height of the reaction solution in the reactor 10 at 1.4 m, a reactor discharge pipe 11 is installed in a side portion of the reactor 10 at a position corresponding to a surface height of the reaction solution, and thus, the reaction solution overflowed into the precipitation tank 20 through the reactor discharge pipe 11. In this case, the reactor discharge pipe 11 was connected to the precipitation tank 20, had a downward slope of 10° in the direction of the precipitation tank 20, and was not provided with a control valve. In addition, operating pressures of the reactor 10 and the precipitation tank 20 were equally controlled to be 40 kg/cm².g.

Under the condition of controlling a temperature of the precipitation tank 20 to 50°C, the polymer contained in the reaction solution was precipitated in a solid state and removed, and a supernatant containing the reaction solution from which the polymer was removed was formed. A precipitation tank discharge pipe 21 was installed at a height at which the supernatant was formed, and the reaction solution from which the polymer was removed was transferred to a storage container 30 through the precipitation tank discharge pipe 21. In this case, the precipitation tank discharge pipe 21 was installed at a position lower than the height of the reactor discharge pipe 11 connected to the precipitation tank 20, and the precipitation tank discharge pipe 21 was provided with the control valve 22. In addition, the operating pressure of the storage container 30 was controlled to be lower than the operating pressure of the precipitation tank 20 to be 37 kg/cm².g.

This operation was performed for 10 hours, and after 10 hours operation, the height of the reaction solution in the reactor 10 was measured. As a result, it could be confirmed that the height of the reaction solution in the reactor 10 was maintained at 1.4 m. This may mean that the control valve 22 provided in the precipitation tank discharge pipe 21 is not clogged by the polymer, and thus, the reaction solution is smoothly discharged from the reactor 10 and the height of the reaction solution is maintained. In this case, it could be confirmed that it is possible to continue the operation.

### Comparative Examples

### Comparative Example 1

A reaction solution was transferred according to a process flow diagram illustrated in FIG. 2 below.

An oligomerization reaction of ethylene was performed by supplying a feed stream to the reactor 10.

The reaction solution was supplied to a storage container 30 through a reactor discharge pipe 12 that is installed in the side portion of the reactor 10 and provided with a control valve 13. In this case, the operating pressure of the reactor 10 was controlled to be 30 kg/cm².g, and an operating pressure of the storage container 30 was controlled to be 27 kg/cm².g. In addition, the height of the reaction solution in the reactor 10 was controlled to 1.4 m by using the control valve 13.

This operation was performed for 10 hours, and after 10 hours operation, the height of the reaction solution in the reactor 10 was measured. As a result, it was confirmed that the height of the reaction solution in the reactor 10 was increased to 3.0 m. This means that the reaction solution is not smoothly discharged from the reactor 10 as the control valve 13 provided in the reactor discharge pipe 12 is clogged by the polymer, and thus, the height of the reaction solution is increased. In this case, since it is difficult to continue operation, it was confirmed that the operation stopped and then the washing needs to be performed.

## Claims

1. A system for transferring reaction solution, comprising:
a reactor that receives and reacts a feed stream to form a reaction solution;
a reactor discharge pipe that is provided on a side portion of the reactor at a position corresponding to a surface height of the reaction solution in the reactor and connected to a precipitation tank to transfer the reaction solution from the reactor to the precipitation tank; and
a precipitation tank that precipitates a polymer contained in the reaction solution.

2. The system for transferring reaction solution of claim 1, wherein the reactor discharge pipe does not include a control valve.

3. The system for transferring reaction solution of claim 1, wherein an operating pressure of the reactor and the precipitation tank is the same.

4. The system for transferring reaction solution of claim 1, wherein the reactor discharge pipe is provided to be inclined downward from the reactor to the precipitation tank.

5. The system for transferring reaction solution of claim 4, wherein a slope of the reactor discharge pipe is 5° to 30°.

6. The system for transferring reaction solution of claim 1, wherein an operating temperature of the precipitation tank is 10°C to 90°C.

7. The system for transferring reaction solution of claim 1, wherein an operating pressure of the precipitation tank is 20 kg/cm².g to 50 kg/cm².g.

8. The system for transferring reaction solution of claim 1, further comprising:
a storage container that receives and stores the reaction solution from the precipitation tank.

9. The system for transferring reaction solution of claim 8, wherein a pressure of the storage container is lower than a pressure of the precipitation tank.

10. The system for transferring reaction solution of claim 9, wherein the pressure of the storage container is 1 kg/cm².g to 5 kg/cm².g lower than the pressure of the precipitation tank.

11. The system for transferring reaction solution of claim 8, further comprising:
a precipitation tank discharge pipe that is connected from the precipitation tank to the storage container,
wherein the precipitation tank discharge pipe is formed in a side portion of the precipitation tank at a height at which a supernatant containing the reaction solution in the precipitation tank is formed.

12. The system for transferring reaction solution of claim 11, wherein the precipitation tank discharge pipe includes a control valve.

13. The system for transferring reaction solution of claim 11, wherein the precipitation tank discharge pipe is provided at a position lower than a height at which the reactor discharge pipe is connected to the precipitation tank.

14. The system for transferring reaction solution of claim 1, wherein the reactor is an ethylene oligomerization reactor.
